# EUROPÄISCHE PATENTSCHRIFT

(11) **EP 1 539 922 B1**
(45) Veröffentlichungstag und Bekanntmachung des Hinweises auf die Patenterteilung: **24.10.2007**
(21) Anmeldenummer: 02807962.2
(22) Anmeldetag: 16.09.2002
(51) Int. Cl.: C12M 3/00

(54) **VERFAHREN ZUR KULTIVIERUNG VON ZELLEN, INSBESONDERE MENSCHLICHER ODER TIERISCHER ZELLEN**
METHOD FOR CULTIVATING CELLS, PARTICULARLY HUMAN OR ANIMAL CELLS
PROCEDE DE MISE EN CULTURE DE CELLULES, NOTAMMENT DE CELLULES HUMAINES OU ANIMALES

(43) Veröffentlichungstag der Anmeldung: 15.06.2005
(73) Patentinhaber: Pan-Biotech GmbH, 94501 Aidenbach (DE)
(72) Erfinder: SEIDL, Josef, 94474 Vilshofen a.d. Donau (DE); SCHERZE, Wilhelm, 90765 Fürth (DE)
(74) Vertreter: Benninger, Johannes
(86) Internationale Anmeldenummer: PCT/EP2002/010357
(87) Internationale Veröffentlichungsnummer: WO 2004/033615

(56) Entgegenhaltungen:
- EP-A- 0 224 800
- GB-A- 2 341 611
- US-A- 5 424 209
- US-A- 5 629 202
- US-A- 5 665 599
- US-A1- 2001 039 045
- CAMISARD V ET AL: "Inline characterization of cell concentration and cell volume in agitated bioreactors using in situ microscopy: Application to volume variation induced by osmotic stress." BIOTECHNOLOGY AND BIOENGINEERING, Bd. 78, Nr. 1, 5. April 2002 (2002-04-05), Seiten 73-80, XP002222991 ISSN: 0006-3592

## Beschreibung

Die Erfindung betrifft ein Verfahren zur Kultivierung von Zellen verschiedenster Art, insbesondere menschlicher oder tierischer Zellen, wobei von Zellen wenigstens einer bestimmten Art jeweils eine Kultur in einer definierten Umgebung angesetzt wird und wobei die Zellen der betreffenden Kultur mit zugeordneten, flüssigen Nährmedien, Wachstumsfaktoren, Gasen oder dergleichen versorgt werden.

Kulturen der vorgenannten Art werden im allgemeinen von einzelnen Zellen angesetzt, die entweder von Gewebeteilen, von primären Kulturen, von Zell-Linien oder Zell-Stämmen durch enzymatische, mechanische oder chemische Zerteilung herrühren.

Bei bisher bekannten Zellkultivierungsverfahren werden zum Ansetzen der Kulturen in der Regel aus Kunststoff bestehende Kulturgefäße verwendet, die in CO₂-Brutschränken inkubiert werden. Diese garantieren eine konstante Temperatur (z.B. 37°C) und eine Pufferung des Mediums durch eine 5 %- bis 10 %-ige CO₂-Begasung. Die Sauerstoffversorgung erfolgt durch einfache Diffusion. Bei den bekannten Verfahren und Einrichtungen zur Kultivierung von Zellen sind Co-Kultivierung und frei veränderliche Inkubationsbedingungen in der Regel nicht möglich.

Zur mikroskopischen Beobachtung oder zu speziellen Untersuchungen müssen die Kulturgefäße aus dem jeweiligen Brutschank entnommen werden, wobei die Inkubation unterbrochen wird, die Zellen sich abkühlen und somit die Versuchsbedingungen nicht mehr konstant sind.

Die bisher bekannten Verfahren zur Kultivierung von Zellen werden jedoch den Anforderungen der modernen Zellkulturtechnologie nicht mehr gerecht.

Insbesondere im Hinblick auf aktuelle Forschungsschwerpunkte in der Pharmaindustrie, die in den Bereichen Entzündung (Rheuma), Krebsbekämpfung, Herz/Kreislauf-Erkrankungen, Aids, Apoptose (programmierter Zelltod) und Blutgerinnung liegen, ist die Entwicklung und Erprobung entsprechender neuer Wirkstoffe und Medikamente mit Hilfe eines neuen Verfahrens zur Kultivierung von Zellen unabdingbar, das es ermöglicht, die Substanz- und Wirkungstestung unter nahezu in-vivo-Bedingungen, d.h., mit nahezu perfekter Abbildung komplexer biologischer Systeme, vor Übertritt in die klinischen Phasen (Testung an Probanten) durchzuführen.

Mit Rücksicht auf die wie oben geschilderte Situation besteht die Forderung nach einer Möglichkeit der Simulation von Reaktionsabläufen innerhalb eines oder mehrerer Organsysteme (z.B. durch Serienschaltung von Zellkulturkammern mit Hepatozyten und anderen Zellarten, Untersuchung auf Abbauprodukte und Metabolite), damit zum einen die Zeiträume zwischen Substanzwirkungserkennung und Arzneimittelzulassung erheblich minimiert werden und zum anderen vor dem Eintritt in die klinische Testphase die notwendigen Erkenntnisse über den Wirkungsmechanismus der Substanz innerhalb eines komplexen biologischen Systems erlangt werden können.

Eine ähnliche Situation liegt beispielsweise auch im Bereich der Kosmetikindustrie vor.

Im Stand der Technik sind beispielsweise multivalente Zellkultursysteme (vgl. z.B. DE 199 15 178 A1), problemadaptierte Zellkultursysteme für spezifische Aufgabenstellungen (vgl. z.B.

WO 98/17822, Verfahren zur Repli.kation von Zellkulturen (vgl. z.B. WO 97/37001), oder automatisierte Zellkulturverfahren (US 5 424 209) bekannt.

Ferner ist beispielsweise aus der WO 99/23206 ein Verfahren zum Mischen einer varizella-infizierten Zellkultur in Rollflaschen bekannt.

Schließlich sind aus der EP 0 999 266 A1 ein Verfahren und eine Vorrichtung zur Aufnahme einer Zellkultur bekannt, wodurch möglichst homogene Bedingungen für die molekularbiologische oder gentechnische Untersuchung von Zellen geschaffen werden sollen.

Mit Rücksicht auf die eingangs geschilderte Situation auf dem Gebiet der modernen Zellkulturtechnologie liegt der vorliegenden Erfindung nunmehr die Aufgabe zugrunde, ein neues, verbessertes Verfahren zur Kultivierung von Zellen verschiedenster Art; insbesondere menschlicher oder tierischer Zellen zu schaffen, das die Nachteile der bisher bekannten Verfahren beseitigt und insbesondere die Möglichkeit bietet, hochkomplexe, biologische Vorgänge in Echtzeit und unter nahezu in-vivo-Bedingungen (d.h. wie im lebenden Organismus) zu simulieren, wobei letztendlich ein computergesteuerter Verfahrensablauf realisierbar sein soll.

Ausgehend von einem Verfahren zur Kultivierung von Zellen verschiedenster Art, insbesondere menschlicher oder tierischer Zellen, wobei von Zellen wenigstens einer bestimmten Art jeweils eine Kultur in einer definierten Umgebung angesetzt wird und die Zellen der betreffenden Kultur mit zugeordneten, flüssigen Nährmedien, Wachstumsfaktoren, Gasen und dergleichen versorgt werden, wird die wie vorstehend definierte Aufgabe erfindungsgemäß durch die Kombination folgender Verfahrensschritte gelöst:
a) Ansetzen wenigstens einer Zellkultur innerhalb wenigstens einer Zellkulturkammer eines Zellkultursystems;
b) Ingangsetzen eines Flusses frei wählbarer, definierter, flüssiger Medien in die wenigstens eine Zellkulturkammer zur kontinuierlichen Versorgung der wenigstens einen Zellkultur;
c) Ingangsetzen eines Stromes unterschiedlicher Gase mit frei wählbaren Konzentrationen in die wenigstens eine Zellkulturkammer zur konstanten, kontinuierlichen Begasung der wenigstens einen Zellkultur;
d) geregeltes bzw. gesteuertes Beheizen der wenigstens einen Zellkulturkammer in der Art und Weise, daß hierin eine konstante Temperatur während der Dauer eines Versuches gewährleistet wird;
e) permanentes mikroskopisches Beobachten der wenigstens einen Zellkultur innerhalb der wenigstens einen Zellkulturkammer, ohne während der Dauer eines Versuches Proben der Zellkultur zu entnehmen; und
f) permanentes Messen sämtlicher relevanten Zellkulturparameter mittels entsprechender, in die wenigstens eine Zellkulturkammer integrierter Sensoren.

Bei dem erfindungsgemäßen Verfahren wird in bevorzugter Weise innerhalb einer vorgegebenen Anzahl von Zellkulturkammern die entsprechende Anzahl von Zellkulturen gleichzeitig angesiedelt und es werden sodann die weiteren Verfahrensmaßnahmen, wie oben definiert, durchgeführt.

Hierbei können die Zellkulturkammern entweder in Reihe oder parallel geschaltet werden.

Durch das erfindungsgemäße Verfahren zur Kultivierung von Zellen ist insbesondere gewährleistet, daß die Zellen sämtlicher Kulturen mit flüssigen Nährmedien, Wachstumsfaktoren, Gasen oder dergleichen kontinuierlich versorgt werden, ohne daß die Zellen einer Kultur ihrer gewohnten, definierten Umgebung entnommen werden müssen, während gleichzeitig sämtliche Zellkulturen ohne Unterbrechung der Begasung permanent mikroskopisch beobachtet werden können.

Gemäß weiterer Ausgestaltung des erfindungsgemäßen Verfahrens können während der Dauer eines Versuchs die Art der flüssigen Medien und/oder deren Strömungsrichtungen und/oder deren Verteilung und/oder deren Durchflußmengen variiert werden, es können aber auch die Art der Gase und/oder deren Strömungsrichtungen und/oder deren Verteilung und/oder die Begasungskonzentrationen variiert werden, was letztendlich dazu führt, daß das erfindungsgemäße Verfahren außerordentlich flexibel gestaltet werden kann.

Insbesondere können im Falle von in Reihe geschalteten Zellkulturkammern eines Zellkultursystems die flüssigen Medien von Zellkulturkammer zu Zellkulturkammer kontinuierlich weitergeleitet werden.

In entsprechender Weise können auch die Gase von Zellkulturkammer zu Zellkulturkammer kontinuierlich weitergeleitet werden.

Um bei dem erfindungsgemäßen Verfahren zur Zellkultivierung während der Dauer eines Versuches in den einzelnen Zellkulturkammern konstante Temperaturen zu gewährleisten, werden die in den einzelnen Zellkulturen herrschenden Temperaturen permanent gemessen und als Temperatur-Istwerte einem entsprechenden Temperaturregel- bzw. Temperatursteuerkreis eingegeben, so daß die Beheizung der jeweiligen Zellkulturkammer entsprechend geregelt bzw. gesteuert werden kann.

Wie weiter unten im einzelnen noch näher erläutert wird, weist zu diesem Zweck jede einzelne Zellkulturkammer eine eigene Heizung auf, während oberhalb der betreffenden Zellkulturkammer jeweils ein Infrarot-Temperaturmesser angeordnet ist, der die in der betreffenden Zellkultur herrschende Temperatur mißt und diesen Temperaturmeßwert an ein Überwachungs- und Steuerungssystem meldet. Ändert sich die anfangs vorgegebene Temperatur in der wenigstens einen Zellkulturkammer, dann wird durch den Temperaturregel- bzw. Steuerkreis bewirkt, daß die Heizleistung der jeweiligen Zellkulturkammerbeheizung vermindert bzw. erhöht wird. Die Temperaturmessung kann aber auch mit Hilfe anderer Temperatursensoren erfolgen.

Wie ebenfalls weiter unten noch näher erläutert wird, sind aus Flexibilitätsgründen die Temperaturen in den einzelnen Zellkulturkammern durch das Überwachungs- und Steuerungssystem während der gesamten Versuchsdauer frei einstellbar und veränderbar.

Eine weitere, besonders vorteilhafte Verfahrensausgestaltung besteht darin, daß innerhalb wenigstens einer Zellkulturkammer zu beiden Seiten einer dort installierten gasdurchlässigen Membran je eine Zellkultur unterschiedlicher Art zum Zwecke einer direkten Co-Kultivierung beider Zellkulturen angesetzt wird.

Eine derartige Co-Kultivierung erfolgt in bevorzugter Weise dadurch, daß die auf der einen Seite der Membran, d.h. auf der apikalen Seite wachsenden Zellen der ersten Zellkultur durch einen ersten Medienfluß versorgt werden, wohingegen die auf der anderen Seite der Membran, d.h. der basolateralen Seite wachsenden Zellen der zweiten Zellkultur mit einem gegenüber dem ersten Medienfluß unterschiedlichen, zweiten Medienfluß versorgt werden. Somit funktionieren die Zellen auf der apikalen Seite als Deckschicht, während die Zellen auf der basolateralen Seite als Innenzellen funktionieren. Die Zellen der ersten Zellkultur und die Zellen der zweiten Zellkultur weisen hierbei durch die Membran einen recht engen Kontakt zueinander auf, so daß die Möglichkeit besteht, Austauschvorgänge innerhalb der Schichten auf der apikalen Seite und der basolateralen Seite zu untersuchen.

Darüberhinaus besteht noch die Möglichkeit, daß dann, wenn gasdurchlässige Membranen mit unterschiedlichen, wählbaren Porengrößen verwendet werden, ein möglicher Austausch von wirksamen bioaktiven Molekülen (z.B. Wachstumsfaktoren, Hormonen, usw.) im Zuge einer derartigen Co-Kultivierung untersucht werden kann. Solche Untersuchungsmöglichkeiten sind insbesondere bei Gewebeteilen wichtig, die aus verschiedenen Zellarten aufgebaut sind, beispielsweise Übergang Endothelzellen-Fibroblasten (Adern) oder Schleimhautzellen-Fibroblasten (Darm, Magen).

Ferner besteht eine weitere, besonders vorteilhafte Verfahrensausgestaltung in der Anwendung des erfindungsgemäßen Verfahrens zur indirekten Co-Kultivierung, wobei verschiedene biologische Systeme (d.h. Gewebe-/Zellarten) in entsprechenden Zellkulturkammern hintereinander geschaltet werden. Auf diese Weise lassen sich ganze Organsysteme gleichsam nachbauen und die entsprechenden Stoffwechselvorgänge untersuchen. Diese Maßnahmen lassen sich durch ein Beispiel näher erläutern: Ein an sich ungiftiger Stoff wird über den Verdauungstrakt aufgenommen und gelangt über den Blutstrom in die Leber. Die Leberzellen bauen den Stoff in Abbauprodukte um, die unter Umständen toxisch wirken können. Um dies zu überprüfen, wird die "verdächtige" Substanz in eine Inkubationskammer eingegeben, die mit Hepatozyten (Leberzellen) besiedelt ist. Über eine definierte Nährmedienversorgung (Medienfluß = "Ader") gelangen evtl. toxische Abbauprodukte in eine sich anschließende Zellkulturkammer, so daß dort z.B. aus absterbenden Nervenzellen auf eine neurotoxische Substanz geschlossen werden kann.

Gemäß einer weiteren, außerordentlich vorteilhaften Ausgestaltung des erfindungsgemäßen Verfahrens ist ein videounterstütztes mikroskopisches Beobachten der wenigstens einen Zellkultur in der wenigstens einen Zellkulturkammer vorgesehen, wie dies ebenfalls weiter unten noch näher erläutert wird.

Das erfindungsgemäße Verfahren läßt sich auch noch dahingehend weiter ausgestalten, daß sämtliche Daten, die gewonnen werden durch
- permanentes mikroskopisches Beobachten der wenigstens einen Zellkultur innerhalb der wenigstens einen Zellkulturkammer und/oder
- permanentes Messen der relevanten Zellkulturparameter und/oder
- permanentes Messen der in der wenigstens einen Zellkultur innerhalb der wenigstens einen Zellkulturkammer herrschenden Temperatur,
zu einem computergesteuerten Überwachungs- und Steuerungssystem zur dortigen Weiterverarbeitung übertragen werden.

Hierbei erfolgt insbesondere das permanente Messen der relevanten Zellkulturparameter mit Hilfe eines software-unterstützten Meßverfahrens.

Eine kontinuierliche Messung von Zellkulturparametern läßt sich vorzugsweise durch spezielle Sonden bzw. Sensoren, beispielsweise für pH-Wert, Lactat, Elektropotential und dergleichen mehr, durchführen, wobei diese Messungen durch eine entsprechende Software ausgewertet und dargestellt werden können. Diese Art der Messung liefert gegenüber herkömmlichen Methoden exaktere Ergebnisse, wodurch bestimmte Fragestellungen analysiert werden können, die mit bisher verwendeten Meßverfahren nicht durchführbar sind. Mit Hilfe eines solchen software-unterstützten Meßverfahrens lassen sich beispielsweise bestimmte Tierversuche in der präklinischen Phase größtenteils ersetzen.

Die Erfindung wird nunmehr nachfolgend anhand von Ausführungsbeispielen näher erläutert, wobei zeigen:
Figur 1 eine schematische Ansicht eines zur Durchführung des erfindungsgemäßen Verfahrens zur Kultivierung von Zellen dienenden, kompletten, geschlossenen Zellkultursystems, bei dem eine vorgegebene Anzahl von Zellkulturkammern zum Einsatz gelangt; und
Figur 2 eine schematische Darstellung einer Hintereinanderschaltung von Zellkulturkammern des in Figur 1 dargestellten Zellkultursystems.

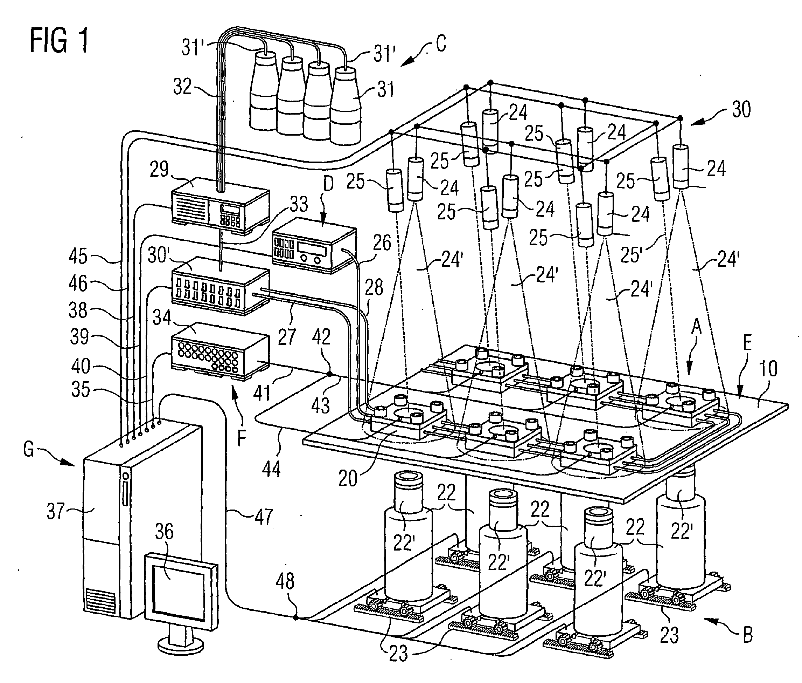

Figur 1 zeigt ein geschlossenes Zellkultursystem 30, bei dem beispielsweise sechs Zellkulturkammern 20 als Gruppe A auf einer entsprechend zugeordneten Basis 21 plaziert sind. Insbesondere bildet die Basis 21 ein Heizsystem E für die Inkubierung, das während der Betriebszeit des Zellkultursystems 30 konstante Temperaturen innerhalb jeder der Zellkulturkammern 20 der Zellkulturkammergruppierung A gewährleistet.

Vorzugsweise erfolgt mit Hilfe dieses Heizsystems E eine elektrische Beheizung der jeweiligen Zellkulturkammer 20, wodurch eine sehr genaue Temperaturregelung ermöglicht ist. Dieses Heizsystem E ist insbesondere in der Weise ausgelegt, daß jede einzelne Zellkulturkammer 20 der Zellkulturkammergruppierung A über ihre eigene Heizung verfügt, die in der Basis 21 integriert ist.

Mit besonderem Vorteil ist das Heizsystem E über eine zugeordnete Software steuerbar. Zu diesem Zweck ist oberhalb der Zellkulturkammergruppierung A ein System aus Infrarot-Temperaturmessern 25 installiert, in der Art, daß jeder einzelnen Zellkulturkammer 20 ein entsprechender Infrarot-Temperaturmesser 25 zugeordnet ist. Der jeweilige Infrarot-Temperaturmesser 25 fühlt mit Hilfe eines von der jeweiligen Zellkulturkammer 20 ausgehenden Infrarotstrahls 25' die in der Zellkultur vorherrschende Temperatur ab und meldet das entsprechende Meßergebnis permanent an ein computergesteuertes Überwachungs- und Steuerungssystem G, das im wesentlichen aus einer Datenverarbeitungsanlage 37 und einem zugehörigen Monitor 36 besteht. Die einzelnen Infrarot-Temperaturmesser 25 sind über eine gemeinsame Verbindungsleitung 45 an das Überwachungs- und Steuerungssystem G angeschlossen. Wenn sich die anfangs vorgegebenen Temperaturen in den Zellkulturkammern 20 der Zellkulturkammergruppierung A ändern, erfolgt automatisch über das Überwachungs- und Steuerungssystem G eine Steuerung bzw. Regelung des Heizsystems E, d.h., die in der einzelnen Zellkulturkammer 20 herrschende Temperatur wird permanent auf eine konstante Temperatur eingeregelt.

Anstatt mittels Infrarot-Temperaturmessern 25 könnte die Temperaturmessung in der einzelnen Zellkulturkammer 20 aber auch mit Hilfe anderer Temperatursensoren durchgeführt werden.

Darüberhinaus kann mit Hilfe der in dem Überwachungs- und Steuerungssystem G enthaltenen Software ermöglicht werden, daß die Temperaturen in den einzelnen Zellkulturkammern 20 der Zellkulturkammergruppierung A während der gesamten Versuchsdauer frei einstellbar und wählbar sind, falls dies aus bestimmten Gründen erforderlich sein sollte.

Zum Zwecke einer permanenten, videogestützten mikroskopischen Beobachtung des Inneren der jeweiligen Zellkulturkammer 20 ist ein Videosystem B mit einem entsprechend zugeordneten Mikroskopsystem vorgesehen. Dieses Videosystem B wird im folgenden näher erläutert.

Unterhalb jeder einzelnen Zellkulturkammer 20 der Zellkulturkammergruppierung A, die im vorliegenden Ausführungsbeispiel insgesamt sechs Zellkulturkammern aufweist, ist eine Videokamera 22 mit Mikroskopaufsatz 22' auf einem mechanisch einstellbaren Fahrtisch 23 angeordnet, somit insgesamt sechs Videokameras 22 mit zugehörigem Mikroskopaufsatz 22'. Somit beobachtet je eine Videokamera 22 mit Mikroskopaufsatz 22' je eine Zellkulturkammer 20. Nach Versuchsstart und nachdem sich aussagekräftige Bereiche in der jeweiligen in der Zellkulturkammer 20 enthaltenen Zellkultur abzeichnen, wird ein Beobachtungssektor in der Zellkulturkammer 20 festgelegt. Dieser Beobachtungssektor wird sodann durch den mechanisch einstellbaren Fahrtisch 23 mittels (nicht dargestellter) Einstellschrauben angefahren, sodann wird der Fahrtisch 23 arretiert und das Videosystem B bleibt infolgedessen während der gesamten Versuchsdauer in der gleichen Position. Ferner wird bei Versuchsstart die Schärfe der Einstellung am jeweiligen Mikroskopaufsatz 22' einjustiert. Dieser Justiervorgang am jeweiligen Mikroskopaufsatz 22' erfolgt für sämtliche sechs Zellkulturkammern 20 und bleibt sodann unverändert bis zum Versuchsende.

Vorzugsweise wird auch das Videosystem B über die im Überwachungs- und Steuerungssystem G enthaltene Software gesteuert. Hierbei wird jede einzelne Videokamera 22 mit Mikroskopaufsatz 22' gesteuert. Dies erfolgt insbesondere in der Art, daß in frei wählbaren Zeitintervallen (beispielsweise im Minutentakt) Bilder von der jeweiligen Zellkultur in der Zellkulturkammer 20 aufgenommen werden, wobei zu dem jeweiligen Zeitpunkt einer solchen Aufnahme eine oberhalb der jeweiligen Zellkulturkammer 20 angeordnete Lichtquelle 24 die entsprechende Zellkultur beleuchtet, so daß eine ausreichende Ausleuchtung des Inneren der Zellkulturkammer 20 für die Videoaufnahmen gewährleistet ist. Wenn die Videoaufnahme beendet ist, bringt die Steuerung die jeweilige Lichtquelle 24 in einen schwach dimmenden Standby-Zustand, bis die nächste Videoaufnahme gemacht wird. Der von einer jeden Lichtquelle 24 ausgehende Lichtstrahl bzw. Lichtkegel, der in das Innere einer jeweiligen Zellkulturkammer 20 durch eine entsprechende (nicht dargestellte) Glasscheibe eintritt, ist in Figur 1 mit 24' bezeichnet.

Sämtliche Lichtquellen 24 sind über eine gemeinsame Verbindungsleitung 46 an das Überwachungs- und Steuerungssystem G angeschlossen.

Durch jeden einzelnen Lichtstrahl bzw. Lichtkegel 24 wird die jeweilige, in der Zellkulturkammer 20 enthaltene Zellkultur flächendeckend ausgeleuchtet.

Das Videosystem B ist ebenfalls über eine Leitung 47 an das Überwachungs- und Steuerungssystem G angeschlossen, wobei von diesem aus die Leitung 47 zu einem Knotenpunkt 48 führt, mit dem die einzelnen Videokameras 22 über entsprechend zugeordnete Leitungen verbunden sind.

Das wie oben erläuterte Videosystem B mit Mikroskopsystem stellt nur eine Ausführungsmöglichkeit dar. Eine mögliche andere Ausführungsform eines solchen Systems zur permanenten Beobachtung des Inneren der Zellkulturkammern besteht darin, daß ein einziges Beobachtungssystem, bestehend aus Videokamera und Mikroskopaufsatz, auf einem Fahrtisch installiert wird und daß dieser Fahrtisch die sechs Zellkulturkammern 20 der Zellkulturkammergruppierung A in frei wählbaren Intervallen abfährt. Die Justierung des Beobachtungssystems erfolgt für die einzelne Zellkultur bei Versuchsstart, d.h., vorzugsweise dann, nachdem sich aussagekräftige Bereiche in der jeweiligen Zellkultur abzeichnen, durch die entsprechende, im Überwachungs- und Steuerungssystem G enthaltene Software, d.h., durch das entsprechende Computerprogramm sind die sechs Anfahrpositionen des Fahrtisches, auf dem das Beobachtungssystem montiert ist, programmiert. Wegen der mechanischen Toleranzen des Fahrtisches muß jedoch ein größerer als der zu beobachtende Bereich innerhalb der einzelnen Zellkulturkammer 20 aufgenommen werden. Innerhalb dieses größeren Bereichs wird nun mittels der Software der zu beobachtende Bereich definiert. Die Software ist in der Lage, Konturen zu speichern und wieder zu erkennen, d.h., beim erneuten Anfahren einer Zellkulturkammer werden die Kontur und die Anordnung der Zellen erkannt und ein anfänglich definierter Beobachtungsbereich gespeichert.

Dieses zuletzt erläuterte Beobachtungssystem ist in den Zeichnungen im einzelnen nicht dargestellt, jedoch erfolgt die Ausleuchtung der einzelnen Zellkulturkammer 20 ebenfalls mit Hilfe der Lichtquellen 24, wie bereits weiter oben im einzelnen erläutert.

Das in Figur 1 dargestellte Zellkultursystem 30 weist ferner noch ein Dosiersystem C für Flüssigkeiten (z.B. flüssige Nährmedien und dergleichen) auf, welches z.B. vier Flüssigkeitsvorratsbehälter 31 mit einer jeweils zugeordneten Flüssigkeitsentnahmeleitung 31' aufweist, wobei sodann aus diesen Flüssigkeitsentnahmeleitungen 31' ein Leitungsbündel 32 gebildet ist. Dieses Leitungsbündel 32 ist andererseits mit einem Pumpensystem 29 verbunden, durch welches die verschiedenen Zellkulturkammern 20 der Zellkulturkammergruppierung A mit frei wählbaren Flüssigkeiten, die in den Flüssigkeitsvorratsbehältern 31 enthalten sind, versorgt werden.

Das Pumpensystem 29 ist seinerseits über eine Leitung 33 an ein Multiventilmodul 30' angeschlossen. Die Zuführung der Flüssigkeiten zu der Zellkulturkammergruppierung A erfolgt von dem Multiventilmodul 30' aus über sterile Schlauchleitungssysteme 27 und 28, wobei diese Flüssigkeiten von den einzelnen Zellkulturkammern 20 flexibel weitergeleitet werden, d.h., von einer Zellkulturkammer zur nächsten. Sowohl die Flüssigkeitszuführung als auch die Flüssigkeitsweiterleitung erfolgt über sterile Schlauchsysteme, die mit Standard-Schlauchverbinderelementen und Verteilern bei Versuchsstart installiert werden, d.h., mit entsprechenden Versorgungskanälen einer jeweiligen Zellkulturkammer 20 verbunden werden. Hierbei wird die Verbindung der Standard-Schlauchelemente (in den Zeichnungen im einzelnen nicht dargestellt) mit den zugeordneten Versorgungskanälen der jeweiligen Zellkulturkammer so aufeinander abgestimmt, daß die Sterilität gewährleistet ist.

Aus Gründen der Flexibilität können die Art der Flüssigkeiten und/oder die Strömungsrichtungen und/oder die Verteilung der Flüssigkeiten und/oder deren Durchflußmengen während des Versuchs geändert bzw. gesteuert werden, wobei eine derartige Steuerung vorzugsweise durch das computergesteuerte Überwachungs- und Steuerungssystem G erfolgt. Zu diesem Zweck sind das Pumpensystem 29 mittels einer Verbindungsleitung 38 und das Multiventilmodul 30' über eine Verbindungsleitung 40 an das Überwachungs- und Steuerungssystem G angeschlossen.

Das Dosiersystem C des Zellkultursystems 30 erlaubt es somit, der Zellkulturkammergruppierung A unterschiedlichste Flüssigkeiten zuzuführen.

Das Zellkultursystem 30 weist darüberhinaus ein Begasungssystem D für unterschiedlichste Gase auf. Dieses Begasungssystem D dient dazu, die verschiedenen Zellkulturkammern 20 der Zellkulturkammergruppierung A mit unterschiedlichen Gasen, z.B. Luft, O₂, N₂, CO₂ zu begasen. Von dem Begasungssystem D aus erfolgt die Gaszuführung zu der Zellkulturkammergruppierung A mittels einer sterilen Schlauchleitung 26. Auch hierbei können die Gase von den verschiedenen Zellkulturkammern 20 unter Verwendung entsprechend zugeordneter Versorgungskanäle flexibel weitergeleitet werden, d.h. von einer Zellkulturkammer zur nächsten.

Gaszuführung und Gasweiterleitung erfolgen insgesamt über sterile Schläuche, die mittels Standard-Schlauchverbinderelementen und Verteilern bei Versuchsstart installiert werden. Die Verbindungen der Schlauchverbinderelemente mit den entsprechend zugeordneten Versorgungskanälen einer jeweiligen Zellkulturkammer 20 sind so aufeinander abgestimmt, daß die Sterilität gewährleistet ist. Auch bei dem Begasungssystem D können aus Flexibilitätsgründen die Art der Gase und/oder die Strömungsrichtungen und/oder die Gasverteilung und/oder die Begasungskonzentration während des Versuchs geändert bzw. gesteuert werden. Zu diesem Zweck ist wiederum das Begasungssystem D über eine Verbindungsleitung 39 an das computergesteuerte Überwachungs- und Steuerungssystem G angeschlossen, das die entsprechende Software für die Steuerung des Begasungssystems D enthält.

Schließlich gehört zu dem Zellkultursystem 30 noch ein Monitoring-System F, das vorgegebene Sensormodule 34 aufweist. Mit Hilfe dieses Monitoring-Systems F können während der gesamten Versuchsdauer die relevanten Parameter in der jeweiligen Zellkulturkammer 20 der Zellkulturkammergruppierung A mittels entsprechend zugeordneter Sensoren gemessen, insbesondere permanent gemessen werden, wobei es sich bei diesen Parametern z.B. um pH-Wert, Glucose, Lactat, Sauerstoff, Elektropotential usw. handelt. Zu diesem Zweck steht das Monitoring-System F über eine Leitung 41, über einen Knotenpunkt 42 und von dort aus über weitere Leitungen 43 und 44 und entsprechend zugeordnete Abzweigleitungen mit den einzelnen Zellkulturkammern 20 der Zellkulturkammergruppierung A des Zellkultursystems 30 in Verbindung.

Die von den (nicht gezeigten) Sensoren gemessenen Parameter werden von dem Monitoring-System F über eine Leitung 35 an das computergesteuerte Überwachungs- und Steuerungssystem G zur entsprechenden Weiterverarbeitung weitergeleitet.

Jede Zellkulturkammer 20 weist entsprechende Sensorikanschlußkanäle auf, wie dies weiter unten noch im einzelnen erläutert wird, wobei die Sensoren und die jeweils zugeordneten Kanäle so aufeinander abgestimmt sind, daß die Sterilität gewährleistet ist.

Mit besonderem Vorzug ist das Monitoring-System F in Verbindung mit dem computergesteuerten Überwachungs- und Steuerungssystem G in der Weise ausgelegt, daß das permanente Messen der relevanten Zellkulturparameter mit Hilfe eines software-unterstützten Meßverfahrens erfolgen kann (wie bereits weiter oben erläutert).

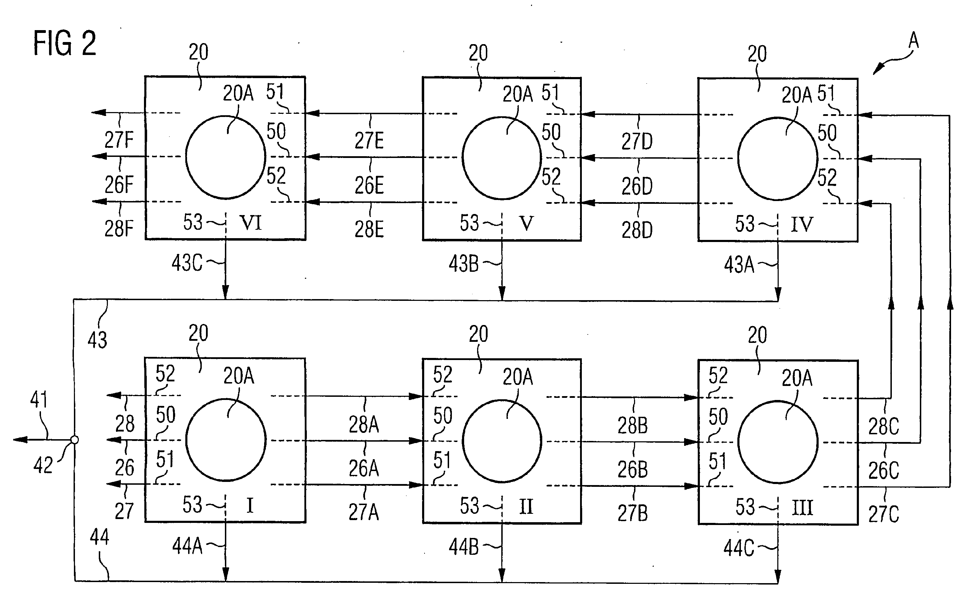

Aus-Figur 2 ist die Zellkulturkammergruppierung A des Zellkultursystems gemäß Figur 1 in schematischer Draufsicht zu ersehen. Bei dieser Zellkulturkammergruppierung A sind insgesamt sechs Zellkulturkammern 20 gleichsam in Reihe geschaltet, derart, daß sowohl die flüssigen Medien als auch die Gase von einer Zellkulturkammer 20 zur anderen, d.h., zur jeweils nachfolgend angeordneten Zellkulturkammer 20 kontinuierlich weitergeleitet werden können.

In jeder der sechs Zellkulturkammern 20 wird mindestens eine zu untersuchende Zellkultur angesiedelt, wobei jedoch im vorliegenden Ausführungsbeispiel der Einfachheit halber von sechs Zellkulturen gesprochen wird, deren jeweiligen Zellen mit definierten flüssigen Nährmedien, Wachstumsfaktoren, Gasen und dergleichen mehr zu versorgen sind.

Zu diesem Zweck wird einerseits ein Fluß frei wählbarer, definierter, flüssiger Medien und andererseits ein Strom unterschiedlicher Gase mit frei wählbaren Konzentrationen in die sechs Zellkulturkammern 20 der Zellkulturkammergruppierung A in Gang gesetzt, wobei, wie bereits weiter oben erläutert, die Zuführung der Flüssigkeiten zu der Zellkulturkammergruppierung A primär von dem Multiventilmodul 30' gemäß Figur 1 aus über die sterilen Schlauchleitungssysteme 27 und 28 erfolgt, während gleichzeitig die Gaszuführung zu der Zellkulturkammergruppierung A von dem Begasungssystem D gemäß Figur 1 aus mittels der sterilen Schlauchleitung 26 erfolgt.

Zur Erleichterung der Übersicht sind die sechs aufeinanderfolgend in Reihe geschalteten Zellkulturkammern 20 mit I, II, III, IV, V und VI gekennzeichnet.

Die Schlauchleitungssysteme 27 und 28 für Flüssigkeiten und die Schlauchleitung 26 für Gase sind unmittelbar mit der ersten Zellkulturkammer I verbunden, derart, daß die Schlauchleitung 26 unmittelbar in einen Gaskanal 50 im Inneren dieser ersten Zellkulturkammer mündet, wohingegen das Schlauchleitungssystem 27 in einen entsprechenden Flüssigkeitskanal 51 und das Schlauchleitungssystem 28 in einen Flüssigkeitskanal 52 jeweils im Inneren dieser ersten Zellkulturkammer I einmünden. Somit wird zunächst die in der ersten Zellkulturkammer I enthaltene Zellkultur mit flüssigen Medien und Gasen versorgt, woraufhin sukzessive die nachfolgenden Zellkulturkammern II bis VI in entsprechender Weise mit flüssigen Medien und Gasen versorgt werden. Im einzelnen ist die Zellkulturkammer I über Flüssigkeits-Schlauchleitungen 27A und 28A und über eine Gas-Schlauchleitung 26A mit der zweiten Zellkulturkammer II verbunden, diese wiederum über Flüssigkeits-Schlauchleitungen 27B und 28B und eine Gas-Schlauchleitung 26B mit der dritten Zellkulturkammer III verbunden, die ihrerseits wiederum über Flüssigkeits-Schlauchleitungen 27C und 28C und eine Gas-Schlauchleitung 26C mit der vierten Zellkulturkammer IV verbunden ist, während diese wiederum über Flüssigkeits-Schlauchleitungen 27D und 28D sowie über eine Gas-Schlauchleitung 26D mit der fünften Zellkulturkammer V verbunden ist, und schließlich ist die letztere über Flüssigkeits-Schlauchleitungen 27E und 28E und über eine Gas-Schlauchleitung 26E mit der sechsten Zellkulturkammer VI verbunden.

Aufgrund dieser Hintereinanderschaltung der sechs Zellkulturkammern 20 mündet jede Flüssigkeits-Schlauchleitung 28A bzw. 28B bzw. 28C bzw. 28D bzw. 28E jeweils in einen Flüssigkeitskanal 52 im Inneren jeder Zellkulturkammer, jede Flüssigkeits-Schlauchleitung 27A bzw. 27B bzw. 27C bzw. 27D bzw. 27E mündet in einen entsprechenden Flüssigkeitskanal 51 im Inneren jeder Zellkulturkammer, wohingegen jede Gas-Schlauchleitung 26A bzw. 26B bzw. 26C bzw. 26D bzw. 26E in einen entsprechenden GasKanal 50 jeder Zellkulturkammer einmündet.

Von der sechsten Zellkulturkammer VI aus gehen Flüssigkeits-Ausgangsleitungen 27F und 28F und eine Gas-Ausgangsleitung 26F ab.

Infolgedessen wird ermöglicht, daß sämtliche Zellkulturen in den sechs Zellkulturkammern I bis VI sowohl mit frei wählbaren, definierten, flüssigen Medien kontinuierlich versorgt werden als auch einer konstanten, kontinuierlichen Begasung durch das Begasungssystem D gemäß Figur 1 unterworfen werden, wie im einzelnen bereits weiter oben erläutert.

Es wird darauf hingewiesen, daß in Fig. 2 lediglich eine von vielen Flußrichtungsmöglichkeiten für Flüssigkeiten und Gase dargestellt ist.

Durch die oben erläuterten, flexiblen Schlauchleitungssysteme für Flüssigkeiten und Gase können auch andere Zellkulturkammer-Kombinationen als die in Fig. 2 gezeigte angesteuert werden.

Von ganz besonderer Bedeutung ist noch, daß die Zellkulturkammergruppierung A insgesamt permanent an das Monitoring-System F gemäß Figur 1 angeschlossen ist, damit während der gesamten Versuchsdauer alle relevanten Parameter in der jeweiligen Zellkulturkammer 20 mittels entsprechend zugeordneter Sensoren gemessen werden können. Jede der sechs Zellkulturkammern 20 ist daher in ihrem Inneren mit einem entsprechenden Kanal 53 für den Sensorikanschluß ausgerüstet. Im einzelnen ist hierbei die erste Zellkulturkammer I über eine Leitung 44A, die zweite Zellkulturkammer II über eine Leitung 44B und die dritte Zellkulturkammer III über eine Leitung 44C mit einer Leitung 44 verbunden, während die vierte Zellkulturkammer IV über eine Leitung 43A, die fünfte Zellkulturkammer V über eine Leitung 43B und die sechste Zellkulturkammer VI über eine Leitung 43C mit einer Leitung 43 verbunden ist. Die Leitungen 43 und 44 führen zu einem Knotenpunkt 42, der über eine Leitung 41 mit dem Monitoring-System F gemäß Figur 1 verbunden ist.

Mit Hilfe der im Inneren einer jeden Zellkulturkammer 20 angeordneten Sensoren, die hier im einzelnen nicht dargestellt sind, ist es möglich, die relevanten Parameter permanent zu messen, wobei sodann die jeweiligen Meßwerte über das Monitoring-System F an das computergesteuerte Überwachungs- und Steuerungssystem G gemäß Figur 1 zur entsprechenden Weiterverarbeitung weitergeleitet werden.

Aus Figur 2 ist noch ersichtlich, daß jede Zellkulturkammer 20 der Zellkulturkammergruppierung A an ihrer Oberseite ein rundes Fenster 20A mit Glasscheibe aufweist, durch das eine flächendeckende Ausleuchtung der in der jeweiligen Zellkulturkammer 20 enthaltenen Zellkultur ermöglicht ist, wie bereits weiter oben anhand der Figur 1 im einzelnen erläutert.

Die Zellkulturkammer als solche bietet im übrigen den Gegenstand einer deutschen Patentanmeldung der gleichen Anmelderin mit der Bezeichnung "Zellkulturkammer für ein Zellkultursystem" (amtliches Aktenzeichen .........).

Mit Hilfe des erfindungsgemäßen Verfahrens zur Kultivierung von Zellen wird insbesondere die Möglichkeit geschaffen, hochkomplexe biologische Vorgänge in Echtzeit und nahezu unter in-vivo-Bedingungen zu simulieren.

Mit ganz besonderem Vorteil läßt sich das erfindungsgemäße Verfahren vor allem zur Erforschung von Zellfunktionen, zur Wirksamkeitsuntersuchung von Medikamenten, zur Arzneimittelentwicklung, zur Co-Kultivierung verschiedener Zelltypen und Gewebeteile, zu organtypischen Studien, zur Beobachtung von Tumorzellen in typischer Umgebung oder zu toxikologischen Studien einsetzen.

## Patentansprüche

1. Verfahren zur Kultivierung von Zellen verschiedenster Art, insbesondere menschlicher oder tierischer Zellen, wobei von Zellen wenigstens einer bestimmter Art jeweils eine Kultur in einer definierten Umgebung angesetzt wird und wobei die Zellen der betreffenden Kultur mit zugeordneten, flüssigen Nährmedien, Wachstumsfaktoren, Gasen und dergleichen versorgt werden, wobei das Verfahren die Kombination folgender Verfahrensschritte umfasst:
a) Ansetzen wenigstens einer Zellkultur innerhalb wenigstens einer Zellkulturkammer (20) eines Zellkultursystems (30);
b) Ingangsetzen eines Flusses frei wählbarer, definierter, flüssiger Medien in die wenigstens eine Zellkulturkammer (20) zur kontinuierlichen Versorgung der wenigstens einen Zellkultur,
c) Ingangsetzen eines Stromes unterschiedlicher Gase mit frei wählbaren Konzentrationen in die wenigstens eine Zellkulturkammer (20) zur konstanten, kontinuierlichen Begasung der wenigstens einen Zellkultur;
d) geregeltes bzw. gesteuertes Beheizen der wenigstens einen Zellkulturkammer (20) in der Art und Weise, dass hierin eine konstante Temperatur während der Dauer eines Versuches gewährleistet wird;
e) permanentes Messen sämtlicher relevanten Zellkulturparameter mittels entsprechender, in die wenigstens eine Zellkulturkammer (20) integrierter Sensoren, **gekennzeichnet, durch**
ein permanentes mikroskopisches Beobachten der Zellkulturkammem (20), ohne während der Dauer eines Versuches Proben der Zellkultur zu entnehmen, wobei eine Kamera mit Mikroskopaufsatz auf einem Fahrtisch die Zellkulturkammem (20) abfährt und die Anfahrpositionen auf einer Software programmiert werden.

2. Verfahren nach Anspruch 1, **dadurch gekennzeichnet, dass** durch die Software Konturen gespeichert werden, wobei beim Anfahren der Kamera diese Konturen erkannt werden.

3. Verfahren nach Anspruch 1 oder 2, **dadurch gekennzeichnet, dass** eine vorgegebene Anzahl von Zellkulturen innerhalb entsprechend zugeordneter Zellkulturkammern (20) angesetzt wird, wobei diese Zellkulturkammern in Reihe geschaltet werden.

4. Verfahren nach Anspruch 1 oder 2, **dadurch gekennzeichnet, dass** eine vorgegebene Anzahl von Zellkulturen innerhalb entsprechend zugeordneter Zellkulturkammern (20) angesetzt wird, wobei diese Zellkulturkammern parallel geschaltet werden.

5. Verfahren nach einem der Ansprüche 1 bis 4, **dadurch gekennzeichnet, dass** während der Dauer eines Versuchs die Art der flüssigen Medien und/ oder deren Strömungsrichtungen und/ oder deren Verteilung und/ oder deren Durchflussmengen variiert werden.

6. Verfahren nach einem der Ansprüche 1, 2, 3 oder 5, **dadurch gekennzeichnet, dass** im Falle von in Reihe geschalteten Zellkulturkammern die flüssigen Medien von Zellkulturkammer zu Zellkulturkammer kontinuierlich weitergeleitet werden.

7. Verfahren nach einem der vorhergehenden Ansprüche, **dadurch gekennzeichnet, dass** während der Dauer eines Versuchs die Art der Gase und/oder deren Strömungsrichtungen und/oder deren Verteilung und/oder die Begasungskonzentrationen variiert werden.

8. Verfahren nach einem der Ansprüche 3, 5, 6 oder 7, **dadurch gekennzeichnet, dass** im Falle von in Reihe geschalteten Zellkulturkammern (20) die Gase von Zellkulturkammer zu Zellkulturkammer kontinuierlich weitergeleitet werden.

9. Verfahren nach einem der vorhergehenden Ansprüche, **dadurch gekennzeichnet, dass** die in der wenigstens einen Zellkultur innerhalb der wenigstens einen Zellkulturkammer (20) herrschende Temperatur permanent gemessen und als Temperatur- Istwert einem entsprechenden Temperaturregel- bzw. Steuerkreis eingegeben wird, wodurch die Beheizung der Zellkulturkammer entsprechend geregelt bzw. gesteuert werden kann.

10. Verfahren nach einem der vorhergehenden Ansprüche, **dadurch gekennzeichnet, dass** innerhalb wenigstens einer Zellkulturkammer (20) zu beiden Seiten einer gasdurchlässigen Membran je eine Zellkultur unterschiedlicher Art zum Zwecke einer direkten Co-Kultivierung beider Zellkulturen angesetzt wird.

11. Verfahren nach Anspruch 10, **gekennzeichnet durch** das Ingangsetzen eines ersten Medienflusses zu der einen Seite der Membran, d.h. der apikalen Seite mit der ersten Zellkultur, und eines gegenüber dem ersten Medienfluss unterschiedlichen, zweiten Medienflusses zu der anderen Seite der Membran, d.h. der basolateralen Seite mit der zweiten Zellkultur.

12. Verfahren nach einem der Ansprüche 1 bis 9, **gekennzeichnet durch** die Anwendung des Verfahrens zur indirekten Co-Kultivierung, wobei verschiedene biologische Systeme (d.h. Gewebe-/ Zellarten) in entsprechenden Zellkulturkammern (20) hintereinander geschaltet werden.

13. Verfahren nach einem der vorhergehenden Ansprüche, **gekennzeichnet durch** ein videounterstütztes mikroskopisches Beobachten der wenigstens einen Zellkultur in der wenigstens einen Zellkulturkammer (20).

14. Verfahren nach einem der vorhergehenden Ansprüche, **dadurch gekennzeichnet, dass** sämtliche Daten, die gewonnen werden durch
- permanentes mikroskopisches Beobachten der wenigstens einen Zellkultur innerhalb der wenigstens einen Zellkulturkammer (20) und/ oder
- permanentes Messen der relevanten Zellkulturparameter und/ oder
- permanentes Messen der in der wenigstens einen Zellkultur innerhalb der wenigstens einen Zellkulturkammer (20) herrschenden Temperatur,
zu einem computergesteuerten Überwachungs- und Steuerungssystem (G) zur dortigen Weiterverarbeitung übertragen werden.

15. Verfahren nach Anspruch 14, **dadurch gekennzeichnet, dass** das permanente Messen der relevanten Zellkulturparameter mit Hilfe eines software- unterstützten Messverfahrens erfolgt.

## Claims

1. A method for cultivating cells of various type, in particular human or animal cells, wherein in each case one culture of cells of at least one specific type is established in a defined environment and wherein the cells of the relevant culture are supplied with assigned, liquid nutrient media, growth factors, gases and the like, wherein the method comprises the combination of the following method steps:
a) establishing at least one cell culture within at least one cell culture chamber (20) of a cell culture system (30);
b) starting a flow of freely selectable, defined, liquid media into the,at least one cell culture chamber (20) so as to provide a continuous supply to the at least one cell culture;
c) starting a flow of different gases with freely selectable concentrations into the at least one cell culture chamber (20) so as to provide constant, continuous gassing of the at least one cell culture;
d) heating the at least one cell culture chamber (20) in a regulated or controlled manner so that a constant temperature is ensured therein over the duration of an experiment;
e) permanently measuring all the relevant cell culture parameters by means of suitable sensors integrated in the at least one cell culture chamber (20),
**characterized by** permanent microscopic observation of the cell culture chambers (20), without samples of the cell culture being taken over the duration of an experiment, wherein a camera with a microscope attachment on a displaceable table moves past the cell culture chambers (10) and the movement positions are programmed on software.

2. The method according to claim 1, **characterized in that** contours are stored by the software, wherein these contours are detected during movement of the camera.

3. The method according to claim 1 or 2, **characterized in that** a predefined number of cell cultures is established within suitably assigned cell culture chambers (20), wherein these cell culture chambers are connected in series.

4. The method according to claim 1 or 2, **characterized in that** a predefined number of cell cultures is established within suitably assigned cell culture chambers (20), wherein these cell culture chambers are connected in parallel.

5. The method according to any one of claims 1 to 4, **characterized in that** the type of liquid media and/or the flow directions thereof and/or the distribution thereof and/or the throughflow volumes thereof are varied over the duration of an experiment.

6. The method according to any one of claims 1, 2, 3 or 5, **characterized in that**, in the case of cell culture chambers connected in series, the liquid media are continuously passed on from cell culture chamber to cell culture chamber.

7. The method according to any one of the preceding claims, **characterized in that** the type of gases and/or the flow directions thereof and/or the distribution thereof and/or the gassing concentrations are varied over the duration of an experiment.

8. The method according to any one of claims 3, 5, 6 or 7, **characterized in that**, in the case of cell culture chambers (20) connected in series, the gases are continuously passed on from cell culture chamber to cell culture chamber.

9. The method according to any one of the preceding claims, **characterized in that** the temperature prevailing in the at least one cell culture within the at least one cell culture chamber (20) is permanently measured and is input as an actual temperature value into a suitable temperature regulating or control circuit, as a result of which the heating of the cell culture chamber can be suitably regulated or controlled.

10. The method according to any one of the preceding claims, **characterized in that** a respective cell culture of different type is established on both sides of a gas-permeable membrane within at least one cell culture chamber (20) for the purpose of a direct co-cultivation of the two cell cultures.

11. The method according to claim 10, **characterized by** the starting of a first media flow to one side of the membrane, i.e. the apical side with the first cell culture, and of a second media flow different from the first media flow to the other side of the membrane, i.e. the basolateral side with the second cell culture.

12. The method according to any one of claims 1 to 9, **characterized by** the use of the method for indirect co-cultivation, wherein different biological systems (i.e. tissue/cell types) are connected one behind the other in suitable cell culture chambers (20).

13. The method according to any one of the preceding claims, **characterized by** a video-assisted microscopic observation of the at least one cell culture in the at least one cell culture chamber (20).

14. The method according to any one of the preceding claims, **characterized in that** all the data, which are obtained by
- permanent microscopic observation of the at least one cell culture within the at least one cell culture chamber (20) and/or
- permanent measuring of the relevant cell culture parameters and/or
- permanent measuring of the temperature prevailing in the at least one cell culture within the at least one cell culture chamber (20),
are transmitted to a computer-controlled monitoring and control system (G) for further processing there.

15. The method according to claim 14, **characterized in that** the permanent measurement of the relevant cell culture parameters takes place by means of a software-assisted measurement method.

## Revendications

1. Procédé pour la culture de cellules des types les plus diversifiés, en particulier de cellules humaines ou animales, étant donné que, pour des cellules d'au moins un type précis, on prépare chaque fois une culture dans un environnement défini, et étant donné que les cellules de la culture concernée sont approvisionnées avec des fluides nutritifs, facteurs de croissance, gaz et autres semblables, étant donné que le procédé comprend la combinaison des étapes suivantes :
a) préparation d'au moins une culture cellulaire à l'intérieur d'au moins une chambre de culture cellulaire (20) d'un système de culture cellulaire (30) ;
b) mise en route dans la ou les chambre(s) de culture cellulaire (20) d'un flux de fluides définis, pouvant être choisis librement, pour l'approvisionnement continu de la ou des culture(s) cellulaire(s) ;
c) mise en route dans la ou les chambre(s) de culture cellulaire (20) d'un courant de gaz différents avec des concentrations pouvant être choisies librement pour assurer une alimentation en gaz constante et continue de la ou des culture(s) cellulaire(s) ;
d) chauffage régulé et commandé de la ou des chambre(s) de culture cellulaire (20) de telle sorte qu'une température constante y soit garantie pendant la durée d'un essai;
e) mesure permanente de tous les paramètres de culture cellulaire importants au moyen de capteurs intégrés correspondants dans la ou les chambre(s) de culture cellulaire (20),
**caractérisée par** une observation microscopique permanente des chambres de culture cellulaire (20) sans prélèvement d'échantillons de la culture cellulaire pendant la durée d'un essai, étant donné qu'une caméra avec microscope rapporté se déplace sur une table de conduite dans les chambres de culture cellulaire (20) et que les positions à parcourir sont programmées dans un logiciel.

2. Procédé selon la revendication 1, **caractérisé en ce que** le logiciel enregistre des contours, étant donné que ces contours sont saisis tors du parcours de la caméra.

3. Procédé selon la revendication 1 ou 2, **caractérisé en ce qu'**on prépare un nombre prédéfini de cultures cellulaires à l'intérieur de chambres de culture cellulaire (20) qui leur sont affectées en conséquence, étant donné que ces chambres de culture cellulaire sont montées en série.

4. Procédé selon la revendication 1 ou 2, **caractérisé en ce qu'**on prépare un nombre prédéfini de cultures cellulaires à l'intérieur de chambres de culture cellulaire (20) correspondantes qui leur sont affectées, étant donné que ces chambres de culture cellulaire sont montées en parallèle.

5. Procédé selon l'une quelconque des revendications 1 à 4, **caractérisé en ce que**, pendant la durée d'un essai, les types de fluides et/ou leurs sens d'écoulement et/ou leur distribution et/ou leurs débits sont variés.

6. Procédé selon l'une quelconque des revendications 1, 2, 3 ou 5, **caractérisé en ce que**, dans le cas de chambres de culture cellulaire montées en série, les fluides sont acheminés de manière continue d'une chambre de culture cellulaire à l'autre.

7. Procédé selon l'une quelconque des revendications précédentes, **caractérisé en ce que**, pendant la durée d'un essai, les types de gaz et/ou leurs sens d'écoulement et/ou leur distribution et/ou les concentrations de gazage sont variés.

8. Procédé selon l'une quelconque des revendications 3, 5, 6 ou 7, **caractérisé en ce que**, dans le cas de chambres de culture cellulaire (20) montées en série, les gaz sont acheminés de manière continue d'une chambre de culture cellulaire à l'autre.

9. Procédé selon l'une quelconque des revendications précédentes, **caractérisé en ce que** la température qui prévaut dans la ou les culture(s) cellulaire(s) à l'intérieur de la ou des chambre(s) de culture cellulaire (20) est mesurée en permanence et est programmée comme valeur effective de température dans un circuit de régulation de température ou circuit de commande correspondant, ce qui permet de réguler ou de commander en conséquence le chauffage de la chambre de culture cellulaire.

10. Procédé selon l'une quelconque des revendications précédentes, **caractérisé en ce que**, à l'intérieur d'au moins une chambre de culture cellulaire (20), on prépare sur chacun des deux côtés d'une membrane perméable aux gaz une culture cellulaire de type différent afin d'assurer une co-culture directe des deux cultures cellulaires.

11. Procédé selon la revendication 10, **caractérisé par** la mise en route d'un premier flux de fluide vers l'un des côtés de la membrane, c'est-à-dire le côté apical avec la première culture cellulaire, et d'un deuxième flux de fluide différent du premier flux de fluide vers l'autre côté de la membrane, c'est-à-dire le côté basolatéral avec la deuxième culture cellulaire.

12. Procédé selon l'une quelconque des revendications 1 à 9, **caractérisé par** l'application du procédé de co-culture indirecte, étant donné que différents systèmes biologiques (c'est-à-dire types de tissus / de cellules) sont montés l'un derrière l'autre dans des chambres de culture cellulaire (20) correspondantes.

13. Procédé selon l'une quelconque des revendications précédentes, **caractérisé par** une observation microscopique assistée par vidéo de la ou des culture(s) cellulaire(s) dans la ou les chambre(s) de culture cellulaire (20).

14. Procédé selon l'une quelconque des revendications précédentes, **caractérisé en ce que** toutes les données qui sont obtenues par
- observation microscopique permanente de la ou des culture(s) cellulaire(s) à l'intérieur de la ou des chambre(s) de culture cellulaire (20) et/ou
- mesure permanente des paramètres de culture cellulaire importants et/ou
- mesure permanente de la température qui prévaut dans la ou les culture(s) cellulaire(s) à l'intérieur de la ou des chambre(s) de culture cellulaire (20) sont transmises à un système de surveillance et de commande (G) commandé par ordinateur pour traitement ultérieur.

15. Procédé selon la revendication 14, **caractérisé en ce que** la mesure permanente des paramètres de culture cellulaire importants se fait à l'aide d'une méthode de mesure assistée par logiciel.
